# EUROPÄISCHE PATENTSCHRIFT

(11) **EP 0 968 003 B1**
(45) Veröffentlichungstag und Bekanntmachung des Hinweises auf die Patenterteilung: **05.06.2002**
(21) Anmeldenummer: 98916875.2
(22) Anmeldetag: 27.02.1998
(51) Int. Cl.: A61L 2/00

(54) **WASSERLÖSLICHES FESTES KONTAKTLINSENPFLEGEMITTEL**
WATER-SOLUBLE SOLID CONTACT LENS CARE PRODUCT
PRODUIT D'ENTRETIEN SOLUBLE SOLIDE POUR LENTILLES DE CONTACT

(30) Priorität: 28.02.1997 DE 19708135; 11.07.1997 DE 19729831
(43) Veröffentlichungstag der Anmeldung: 05.01.2000
(73) Patentinhaber: Sturm, Albert, 50931 Köln (DE)
(72) Erfinder: Sturm, Albert, 50931 Köln (DE)
(74) Vertreter: Meyers, Hans-Wilhelm, Dr.Dipl.-Chem.
(86) Internationale Anmeldenummer: EP9801109
(87) Internationale Veröffentlichungsnummer: WO9837921

(56) Entgegenhaltungen:
- EP-A- 0 209 071
- WO-A-91/12826
- WO-A-97/11722

## Beschreibung

Gegenstand der vorliegenden Erfindung ist ein wasserlösliches, festes Kontaktlinsenpflegemittel gemäß Anspruch 1.

Kontaktlinsen erfreuen sich bei fehlsichtigen Personen großer Beliebtheit. Ein Problem bei der Verwendung von Kontaktlinsen ist nach wie vor deren sachgerechte Pflege. Es sind eine Vielzahl von verschiedenen Systemen bekannt, denen im wesentlichen gemeinsam ist, daß eine Desinfektion der Kontaktlinse nach Tragen der Kontaktlinse, meistens über Nacht, erfolgt, wobei die Desinfektion durch chemische Mittel bewirkt wird, die aufgrund ihres aggressiven Charakters nachträglich neutralisiert werden müssen. Dazu hat sich eine Vielzahl von Pflegemitteln auf dem Markt durchgesetzt, so z. B. eine wäßrige Wasserstoffperoxidlösung als Desinfektionslösung, welche durch nachgeschaltete Stufen neutralisiert oder zersetzt wird. Dazu sind im Stand der Technik verschiedene Systeme bekannt, wie beispielsweise katalytische Zersetzung an Edelmetalloberflächen, enzymatische Zersetzung mittels beispielsweise Katalase sowie chemische Neutralisation durch Reduktion des Wasserstoffperoxids mit Natriumthiosulfat. Auch das Problem der Entfernung von metabolischen Ablagerungen, insbesondere Proteinablagerungen, auf den Linsen oder im Linsenmaterial ist im Stand der Technik grundsätzlich gelöst. Dazu werden beispielsweise proteasehaltige Mittel in einer vorgeschalteten Reinigungsstufe zugefügt. Als Protease, die Proteinablagerungen in den Kontaktlinsenmatrices, sofern weiche Kontaktlinsen betroffen sind, entfernt, ist insbesondere Subtilisin zu nennen. Stellvertretend für den Stand der Technik dient die EP 0 219 220. Dort wird ein kombiniertes Kontaktlinsenpflegemittel beschrieben, welches die Kontaktlinsen sowohl desinfiziert als auch reinigt. Die Desinfektion erfolgt hierbei mittels einer Lösung von Wasserstoffperoxid und einer Protease, die in peroxidhaltiger Lösung aktiv ist. Als entsprechende Protease wird Subtilisin genannt.

Die Entfernung der metabolischen Ablagerungen, insbesondere Proteinablagerungen, in der Kontaktlinsenmatrix ist äußerst wichtig, da diese Ablagerungen zu Veränderungen der Linsenmatrix führen, die von Veränderungen der optischen Eigenschaften bis hin zum Hervorrufen von Entzündungen reichen.

Obwohl verschiedene Systeme bereits am Markt erhältlich sind, scheint die Pflege von insbesondere weichen Kontaktlinsen so aufwendig zu sein, daß man in jüngerer Zeit verstärkt dazu übergegangen ist, sogenannte Einwegkontaktlinsen zu vertreiben. Hierbei steht dann die Reinigung und Desinfektion nicht mehr im Vordergrund, da nach einer bestimmten Tragezeit die Kontaktlinsen einfach weggeworfen werden. Andererseits führt dies jedoch dazu, daß nur noch vorkonfektionierte Massenware angeboten werden kann, da eine individuelle Anpassung der Kontaktlinse mit zu hohen Kosten verbunden ist, so daß eine individuell angepaßte Einmallinse nicht erschwinglich ist. Mit dem Verzicht auf individuelle Anpassungen werden jedoch Kontaktlinsen mit nicht optimalen Eigenschaften an die jeweiligen Patienten ausgegeben, so daß sich aus physiologischer Sicht eine solche Verfahrensweise nicht empfiehlt.

Insbesondere nachteilig am Stand der Technik ist, daß Proteasen üblicherweise in einem gesonderten Reinigungsschritt, der vor der Desinfektion liegt, angewendet werden. Dadurch wird eine erhöhte Bereitschaft des Verwenders gefordert, die Reinigung und Desinfektion sachgerecht durchzuführen. Nach der Reinigung mittels Proteasen wird diese Lösung üblicherweise verworfen und danach eine gesonderte Desinfektionsphase durchgeführt. Dieses zweistufige Verfahren wird von Anwendern als lästig empfunden und fördert durch sorglosen Gebrauch unsachgemäße Verhaltensweisen. So kann es beispielsweise passieren, daß der Desinfektionsschritt vergessen wird und die Linse direkt aus der Proteaselösung aufgesetzt wird. Bei einer anderen Verfahrensweise wird der Desinfektionslösung eine Protease zugegeben, wobei diese allerdings in so hoher Menge beigefügt wird, daß nach dem Reinigungsschritt die Protease, mittels physiologischer Lösung, wieder entfernt werden muß. Anderenfalls würde zuviel Protease in der Kontaktlinsenmatrix verbleiben, was zu Irritationen auf dem Auge des Anwenders führen kann.

Das Dokument WO-A-9112826 beschreibt Tabletten aus Katalase, welche von einer äusseren Schicht aus Subtilisin umgeben ist.

Aufgabe der vorliegenden Erfindung ist es mithin, Systeme bereitzustellen, die es in einfacher Weise erlauben, eine Reinigung und Desinfektion der Kontaktlinsen zu ermöglichen.

Überraschenderweise wird die Aufgabe der vorliegenden Erfindung in einfacher Weise durch ein Kontaktlinsenpflegemittel mit den Merkmalen des Patentanspruchs 1 gelöst. Die Unteransprüche betreffen bevorzugte Ausführungsformen des erfindungsgemäßen Kontaktlinsenpflegemittels.

Das erfindungsgemäße Kontaktlinsenpflegemittel weist mindestens ein erstes und ein zweites Kompartiment auf. Das erste Kompartiment enthält mindestens ein Mittel, insbesondere metabolische Protease, zur Entfernung oder Unterstützung der Reinigung von proteinartigen Ablagerungen auf einer Kontaktlinse. Das mindestens zweite Kompartiment enthält mindestens ein Mittel, das in der Lage ist, eine Kontaktlinsendesinfektionslösung zu neutralisieren. Erfindungswesentlich ist dabei, daß sich das erste Kompartiment schneller in einer Kontaktlinsendesinfektionslösung aufgelöst hat als das zweite Kompartiment. Beide Kompartimente können gleichzeitig der Kontaktlinsendesinfektionslösung ausgesetzt sein indem das erste Kompartiment das zweite Kompartiment nicht allseitig umschließt. Als Mittel zur Entfernung metabolischer Ablagerungen sind Proteasen, insbesondere Proteasen vom Serintyp, geeignet. Insbesondere bevorzugt ist eine Enzymklasse, die als alkalische Proteasen, allgemein als Subtilisin, bekannt sind. Entsprechende Enzyme sind in der EP 0 219 220 charakterisiert.

Das zweite Kompartiment enthält ein Kontaktlinsendesinfektionsneutralisationsmittel, wobei im Falle einer wäßrigen Wasserstoffperoxidlösung Enzyme, wie Katalase, oder Chemikalien, wie Natriumdithionit, zu nennen sind.

Vorzugsweise besteht das erfindungsgemäße Kontaktlinsenpflegemittel aus einem ersten Kompartiment und einem zweiten Kompartiment. Das zweite Kompartiment wird von dem ersten Kompartiment nicht allseitig umschlossen. Dadurch wird der Desinfektionslösung gleichzeitiger Zugang zu den Kompartimenten ermöglicht. In einer besonderen Ausführungsform umschließt das erste Kompartiment das zweite Kompartiment zumindest teilweise konzentrisch. In einer anderen Ausführungsform des erfindungsgemäßen Kontaktlinsenpflegemittels bildet dieses einen Formkörper mit zwei aufeinander senkrecht stehenden Achsen, wobei das erste Kompartiment um eine erste Achse und das zweite Kompartiment um eine zweite Achse angeordnet ist. Die Anordnung der Kompartimenten ist vorzugsweise rotationssymmetrisch um eine Achse.

Das Desinfektionsmittel kommt mit den Kompartimenten in Kontakt und löst das erste Kompartiment schneller auf als das zweite Kompartiment. Die Auflösung des ersten Kompartiments kann durch Additive beschleunigt werden. Als Additive kommen insbesondere Stoffe, die eine leicht lösliche Matrix bilden oder Stoffgemische, die in wäßriger Lösung ein Sprudeln erzeugen, in Betracht. Besonders bevorzugt ist die Verwendung von Additiven, die nach Auflösung der Kompartimente zur Bildung einer isotonischen Lösung beitragen.

Das zweite Kompartiment kann Additive enthalten, die eine Auflösung des zweiten Kompartiments verzögern. Dies kann beispielsweise durch die Verwendung von schwerer löslichen Stoffen oder Stoffgemischen erfolgen als diejenigen, die im ersten Kompartiment verwendet werden.

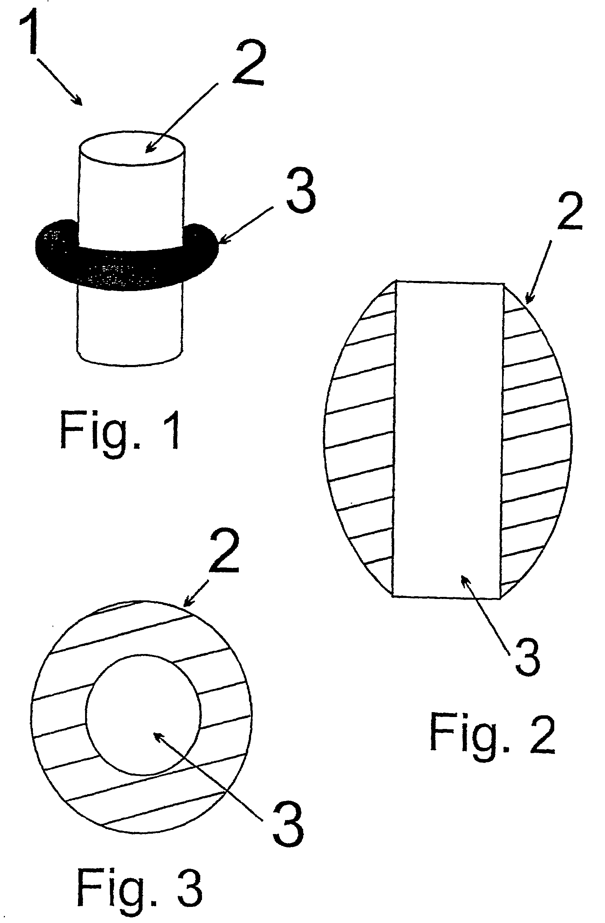

Die Figur 1 zeigt eine bevorzugte Ausführungsform des erfindungsgemäßen Kontaktlinsenpflegemittels. Hierbei ist ein im wesentlichen zylindrisch gestalteter Formkörper das erste Kompartiment 2 enthaltend als Protease Subtilisin. Der im wesentlichen zylindrische Formkörper 2, der das Subtilisin in einer üblichen Tablettenmatrix verpresst enthält, ist umschlossen von dem zweiten Kompartiment 3, und zwar in Form eines Torus, in dessen Zentrum sich der im wesentlichen zylindrische Formkörper 2 des ersten Kompartiments sich befindet.

Figur 2 zeigt eine weitere Ausführungsform, in der das erste Kompartiment zentrisch durchbohrt ist und diese Bohrung mit dem zweiten Kompartiment verfüllt ist

Figur 3 zeigt eine weitere Ausführungsform, in der das erste Kompartiment das zweite vollständig umschließt (nicht unter Anspruch 1 fallend).

Der Anwender füllt zunächst die wäßrige Desinfektionslösung, vorzugsweise eine 3%ige Wasserstoffperoxidlösung in an sich bekannte Reinigungsvorrichtungen, gibt das erfindungsgemäße Kontaktlinsenpflegemittel sowie die zu reinigenden und desinfizierenden Kontaktlinsen hinzu. Die Desinfektionslösung löst dabei das Subtilisin enthaltende Kompartiment auf, so daß die Entfernung der Ablagerungen auf der Linse beginnt. Gleichzeitig wird die Kontaktlinse durch die Einwirkung des Desinfektionsmittels, H₂O₂, desinfiziert. Je nach Ausführungsform mehr oder weniger gleichzeitig wird auch das zweite Kompartiment mit dem Desinfektionsneutralisierungsmittel gelöst. Aufgrund der schnelleren Auflösungskinetik des ersten Kompartiments ist dieses bereits vollständig aufgelöst, wenn das zweite Komparfiment sich erst teilweise in der Desinfektionslösung aufgelöst hat. Die im zweiten Kompartiment enthaltene Katalase wird dann sukzessive weiter in die Desinfektionslösung hineingelöst, so daß dort die Katalase in zunehmenden Maße die Zersetzung des Wasserstoffperoxids bewirkt.

Durch das erfindungsgemäße Kontaktlinsenpflegemittel wird ein neuartiges Kontaktlinsenpflegesystem ermöglicht. Der Anwender kann nämlich eine individuelle Reinigungsprozedur bestimmen, die nach Rücksprache mit dem Kontaktlinsenanpasser auf die Verhältnisse des Anwenders abgestimmt ist. So ist es beispielsweise möglich, die herkömmliche Desinfektion, beispielsweise an zwei aufeinanderfolgenden Tagen durchzuführen, wohingegen das erfindungsgemäße Kontaktlinsenpflegemittel dann am dritten Tag zugegeben wird, um bei moderater Verschmutzung mit Proteinen nicht jeden Tag die Proteinreinigung durchführen zu müssen. Ebenso kann bei stärkerer Verschmutzung bereits jeden zweiten Tag die Proteinablagerungsentfernung durchgeführt werden, unter Einsatz des erfindungsgemäßen Kontaktlinsenpflegemittels. Die Darreichung des erfindungsgemäßen Kontaktlinsenpflegemittels erfolgt beispielsweise in Form von Blisterpackungen, in denen dann auch Tabletten angeboten werden, die Desinfektionsmittelneutralisatoren enthalten. Es kann beispielsweise eine Blisterpackung angeboten werden, in der, beginnend mit dem erfindungsgemäßen Kontaktlinsenpflegemittel in Tablettenform, beispielsweise zwei Tabletten angeboten werden, welche das Mittel, insbesondere die Protease, zur Entfernung von metabolischen Ablagerungen, z.B. Proteinablagerungen, nicht enthält. Erst am vierten Tag wird dann in der Blisterpackung das erfindungsgemäße Kontaktlinsenpflegemittel erscheinen, wiederum gefolgt von zwei Tabletten, die lediglich das Neutralisationsmittel enthalten.

Das erfindungsgemäße Kontaktlinsenpflegemittel kann die in den Kompartimenten befindlichen Stoffe auch in Granulatform beinhalten, wobei die Kompartimente z. b. als Kapseln ausgebildet sind.

Es kann bevorzugt sein, die Entfernung des Wasserstoffperoxids durch variabel vorbestimmte Behältervolumen nicht vollständig durchzuführen. Damit verbleibt in der Aufbewahrungslösung oder Reinigungslösung der Linse ein Rest Wasserstoffperoxidgehalt. Der Vorteil der Variabilität des zu verwendenden Volumens an Wasserstoffperoxid besteht darin, daß bei längerer Aufbewahrungszeit entsprechend mehr H₂O₂-haltige Lösung zur Verfügung gestellt werden kann, so daß die Zersetzung nicht vollständig erfolgt und ein Restgehalt H₂O₂ zur Bereitstellung eines andauerenden bacteriziden bzw. bacteriostatischen Milieus in der Lösung verbleibt. Insbesondere kann dieser Wasserstoffperoxidrestgehalt bis zu 0,8 Gew.-%, bedarfsweise weniger, z. B. bis zu 0,2 Gew.-%, betragen. Dies hat den Vorteil, daß insbesondere bei längerer Aufbewahrungszeit der Linse in der Aufbewahrungslösung eine Nachverkeimung nicht auftritt, da ein anhaltendes bacterizides bzw. bacteriostatisches Milieu anwesend bleibt.

Es kann so eingestellt sein, daß entweder kein oder ein nur sehr geringer Reizwert verbleibt, der selbst bei ausgeprägten Augensensibilität schon durch ehr kurzzeitiges Nachspülen mit geeigneten handelsüblichen Abspül-Kochsalzlösungen beherrschbar ist.

## Patentansprüche

1. Wasserlösliches, festes Kontaktlinsenpflegemittel (1) mit mindestens einem ersten (2) und einem zweiten Kompartiment (3), enthaltend in dem ersten Kompartiment (2) mindestens ein Mittel, insbesondere eine Protease, zur Entfernung oder Unterstützung der. Reinigung von metabolischen Ablagerungen auf einer Kontaktlinse und in dem zweiten Kompartiment (3) mindestens ein Mittel enthält, das in der Lage ist, eine Kontaktlinsendesinfektionslösung zu neutralisieren, **dadurch gekennzeichnet, daß** das erste Kompartiment (2) schneller in der Kontaktlinsendesinfektionslösung aufgelöst ist als das zweite Kompartiment (3) und daß beide Kompartimente (2, 3) gleichzeitig der Kontaktlinsendesinfektionslösung aussetzbar sind, indem das erste Kompartiment (2) das zweite Kompartiment (3) nicht allseitig umschließt.

2. Kontaktlinsenpflegemittel nach Anspruch 1, **dadurch gekennzeichnet, daß** zwei Kompartimente (2, 3) vorliegen.

3. Koatakümsenpflegemittel nach Anspruch 1, **dadurch gekennzeichnet, daß** das erste Kompartiment (2) das zweite Kompartiment (3) konzentrisch umschließt.

4. Kontaktlinsenpflegemittel nach Anspruch 1 bis 3, **dadurch gekennzeichnet, daß** das Kontaktlinsenpflegemittel ein Formkörper mit zwei aufeinander senkrecht stehenden Achsen ist, wobei das erste Kompartiment (2) um die erste Achse und das zweite Kompartiment (3) um die zweite Achse angeordnet ist.

5. Kontaktlinsenpflegemittel nach mindestens einem der Ansprüche 1 bis 4, **dadurch gekennzeichnet, daß** die Auflösung des ersten Kompartiments (2) in der Desinfektionslösung durch Additive beschleunigt wird,

6. Kontaktlinsenpflegemittel nach mindestens einem der Ansprüche 1 bis 5, **dadurch gekennzeichnet, daß** die Auflösung des zweiten Kompartiments (3) in der Desinfektionslösung durch Additive verzögert wird.

7. Kontaktlinsenpflegemittel nach mindestens einem der Ansprüche 1 bis 6, **dadurch gekennzeichnet, daß** das Mittel zur Neutralisierung einer Kontaktlinsendesinfektionslösung nur in einer solchen Menge vorhanden ist, daß eine teilweise Neutralisierung des Kontaktlinsendesinfektionsmittels erfolgt.

8. Kontaktlinsenpflegemittel nach mindestens einem der Ansprüche 1 bis 7, **dadurch gekennzeichnet, daß** das erste Kompartiment (2) Subtilisin und/oder das zweite Kompartiment (3) Katalase enthält.

## Claims

1. A water-soluble and solid contact lens care agent (1) having at least one first (2) and one second compartment (3), containing in said first compartment (2) at least one agent, especially a protease, for the removing or supporting the cleaning of metabolic depositions on a contact lens and in said second compartment (3) at least one agent capable of neutralizing a contact lens disinfection solution, **characterized in that** said first compartment (2) is more quickly dissolved in said contact lens disinfection solution than said second compartment (3), and that both compartments (2, 3) can be simultaneously exposed to said contact lens disinfection solution due to the fact that said first compartment (2) does not enclose said second compartment (3) from all sides.

2. The contact lens care agent according to claim 1, **characterized in that** two compartments (2, 3) are present.

3. The contact lens care agent according to claim 1, **characterized in that** said first compartment (2) encloses said second compartment (3) concentrically.

4. The contact lens care agent according to claims 1 to 3, **characterized in that** said contact lens care agent is a molded part with two mutually perpendicular axes, wherein said first compartment (2) is arranged around the first axis and said second compartment (3) is arranged around the second axis.

5. The contact lens care agent according to at least one of claims 1 to 4, **characterized in that** the dissolution of said first compartment (2) in the disinfection solution is accelerated by additives.

6. The contact lens care agent according to at least one of claims 1 to 5, **characterized in that** the dissolution of said second compartment (3) in the disinfection solution is decelerated by additives.

7. The contact lens care agent according to at least one of claims 1 to 6, **characterized in that** said agent for neutralizing the contact lens disinfection solution is present in as low an amount that said contact lens disinfection agent is but partially neutralized.

8. The contact lens care agent according to at least one of claims 1 to 7, **characterized in that** said first compartment (2) contains subtilisin, and/or said second compartment (3) contains catalase.

## Revendications

1. Produit de soin pour lentilles de contact (1) solide, soluble dans l'eau, ayant au moins un premier (2) et un deuxième compartiments (3), contenant, dans le premier compartiment (2), au moins un produit, en particulier une protéase, permettant d'éliminer ou de favoriser le nettoyage des dépôts métaboliques sur une lentille de contact et, dans le deuxième compartiment (3), au moins un produit en mesure de neutraliser une solution de désinfection de lentilles de contact, **caractérisé en ce que** le premier compartiment (2) est dissout plus rapidement dans la solution de désinfection de lentilles de contact que le deuxième compartiment (3) et que les deux compartiments (2, 3) peuvent être exposés simultanément à la solution de désinfection de lentilles de contact, **en ce que** le premier compartiment (2) n'entoure pas de tous côtés le deuxième compartiment (3).

2. Produit de soin pour lentilles de contact selon la revendication 1, **caractérisé en ce que** l'on a prévu deux compartiments (2, 3).

3. Produit de soin pour lentilles de contact selon la revendication 1, **caractérisé en ce que** le premier compartiment (2) entoure concentriquement le deuxième compartiment (3).

4. Produit de soin pour lentilles de contact selon les revendications 1 à 3, **caractérisé en ce que** le produit de soin pour lentille de contact est un corps profilé ayant deux axes perpendiculaires l'un à l'autre, le premier compartiment (2) étant disposé autour du premier axe et le deuxième compartiment (3) étant disposé autour du deuxième axe.

5. Produit de soin pour lentilles de contact selon au moins l'une des revendications 1 à 4, **caractérisé en ce que** la dissolution du premier compartiment (2) dans la solution de désinfection est accélérée grâce à des additifs.

6. Produit de soin pour lentilles de contact selon au moins l'une des revendications 1 à 5, **caractérisé en ce que** la dissolution du deuxième compartiment (3) dans la solution de désinfection est retardée grâce à des additifs.

7. Produit de soin pour lentilles de contact selon au moins l'une des revendications 1 à 6, **caractérisé en ce que** des produits de neutralisation d'une solution de désinfection de lentilles de contact sont prévus uniquement en une quantité faisant que s'effectue une neutralisation partielle du produit de désinfection pour lentilles de contact.

8. Produit de soin pour lentilles de contact selon au moins l'une des revendications 1 à 7, **caractérisé en ce que** le premier compartiment (2) contient de la subtilisine et/ou le deuxième compartiment (3) contient de la catalase.
